# EUROPÄISCHE PATENTSCHRIFT

(11) **EP 0 557 949 B1**
(45) Veröffentlichungstag und Bekanntmachung des Hinweises auf die Patenterteilung: **16.12.1998**
(21) Anmeldenummer: 93102845.0
(22) Anmeldetag: 24.02.1993
(51) Int. Cl.: C07C 255/50, C07C 253/30

(54) **Verfahren zur Herstellung von 2,4,5-Trifluorbenzonitril**
Process for the preparation of 2,4,5 trifluorobenzonitrile
Procédé pour la préparation du trifluoro 2,4,5 benzonitrile

(30) Priorität: 26.02.1992 DE 4205786
(43) Veröffentlichungstag der Anmeldung: 01.09.1993
(73) Patentinhaber: Clariant GmbH, 65929 Frankfurt am Main (DE)
(72) Erfinder: Pfirmann, Ralf, Dr., W-6103 Griesheim (DE); Papenfuhs, Theodor, Dr., W-6000 Frankfurt am Main 50 (DE)

(56) Entgegenhaltungen:
- EP-A- 0 431 373
- EP-A- 0 433 124
- EP-A- 0 497 239
- US-A- 4 973 772
- CHEMICAL ABSTRACTS, vol. 113, no. 9, 27. August 1990, Columbus, Ohio, US; abstract no. 77918q, Seite 738 ;Spalte 2 ; & CN-A-1 031 074 (TIANJIN RESEARCH INSTITUTE OF MEDICINAL INDUSTRY)

## Beschreibung

Die vorliegende Erfindung betrifft ein neues, verbessertes Verfahren zur Herstellung von 2,4,5-Trifluorbenzonitril, einem wertvollen Zwischenprodukt zur Herstellung von antibakteriellen Mitteln der Fluorchinoloncarbonsäurereihe über das Verseifungsprodukt 2,4,5-Trifluorbenzoesäure.

Bisher kann 2,4,5-Trifluorbenzonitril, das sich nach literaturbekannten Methoden (EP 431 373, EP 433,124, H. Henecka in Houben-Weyl-Müller, Methoden der Organischen Chemie, Bd. 8 (1952), 427-433) zunächst in 2,4,5-Trifluorbenzoesäure und dann in die Wirkstoffe (J.P. Sanchez et al., J. Med. Chem. 31 (1988), 983-991; EP 227 088; DE 3 600 891; DE 3 420 743; JP 60 072 885; EP 191 185) überführen läßt, nur nach technisch unbefriedigenden, wirtschaftlich ungünstigen Verfahren hergestellt werden.

Durch Halex-(Chlor/Fluor-Austausch)-Reaktion von 2,4-Dichlor-5-fluorbenzonitril (EP 431 373) im technisch unerwünschten Lösungsmittel Dimethylsulfoxid unter Verwendung von sprühgetrocknetem Kaliumfluorid erhält man schlechte Ausbeuten (43 %) an 2,4,5-Trifluorbenzonitril. Ferner erhält man 2,4,5-Trifluorbenzonitril als Nebenprodukt in Ausbeuten um 20 % bei der Herstellung von 2-Chlor-4,5-difluorbenzonitril (EP 433 124) oder durch Brom-Cyan-Austausch von 2,4,5-Trifluorbrombenzol mittels Alkalimetallcyaniden (EP 191 195).

2,4,5-Trifluorbenzoesäure kann auch auf anderen Wegen hergestellt werden, die zum Teil durch Vielstufigkeit und schlechte Gesamtausbeuten, zum Teil durch Werkstoff- und reaktionstechnische Schwierigkeiten ungünstig sind. Hierzu zählen die Fluorierung von 2,5-Difluor-4-chlorbenzoylfluorid (PCT WO 90/12 780) und 2,4-Dichlor-5-fluorbenzoylfluorid (JP 01/226 851; DE 3 420 796; EP 164 619) zu 2,4,5-Trifluorbenzoylfluorid mit anschließender Verseifung. Dazu zählt man auch die klassische Fluorierung von 4,5-Difluoranthranilsäure nach der Schiemann-Reaktion zu 2,4,5-Trifluorbenzoesäure (G.C. Finger et al., CA 50 (1955), 9312). Mit Werkstoffproblemen verbunden sind Dehalogenierungsreaktionen von Tetrafluorphthalodinitrilen (EP 307 897, JP 01/160 944) oder -diestern. Diesen Methoden gemeinsam ist eine selektive Decarboxylierung der synthetisierten Trifluor(iso)phthaläsuren, wobei nicht selten höhere, schwer abzutrennende Isomerenrestgehalte auftreten (US 4 935 541, JP 01/052 737; JP 01/025 737). Durch Fluorierung von 3,4,6-Trichlorphthalimiden (EP 431 294) und anschließende Hydrolyse erhaltene Trifluorphthalsäure kann ebenfalls nicht vollständig selektiv zu 2,4,5-Trifluorbenzoesäure decarboxyliert werden.

Wirtschaftlich ungünstig ist die Acylierung von 1,2,4-Trifluorbenzol mittels unter Umständen aliphatisch chlorierter Acerylchloride und anschließender Haloform-Reaktion (EP 411 252, DE 3 840 371, DE 3 840 375, JP 02 184 650), da 1,2,4-Trifluorbenzol selbst durch aufwendige Schritte, wie sie der Schiemann-Reaktion eigen sind, hergestellt werden muß.

Es wurde nun gefunden, daß ein neues Verfahren zur Herstellung von 2,4,5-Trifluorbenzonitril das gewünschte Produkt in guten Ausbeuten liefert. Es ist dadurch gekennzeichnet, daß man 2,4-Dichlor-5-fluorbenzonitril mit etwa 105 bis etwa 150 Mol-%, insbesondere 110 bis 120 Mol-% eines Alkalimetallfluorids oder eines Tetraalkyl-(C₁-C₁₈)-ammoniumfluorids oder Mischungen aus solchen Fluoriden pro auszutauschendem Chloratom in Gegenwart von 0,5 bis 10 Mol-%, bezogen auf 2,4-Dichlor-5-fluorbenzol, eines Tetraalkyl-(C₁-C₁₈)-phosphoniumchlorids oder -bromids, Tetraphenylphosphoniumchlorids oder - bromids, oder (Phenyl)ₘ(alkyl(C₁-C₁₈))ₙ-phosphoniumchlorids oder -bromids, wobei m = 1 bis 3, n = 3 bis 1 und m + n = 4 ist, oder 5 bis 100 Gew.-%, bezogen auf eingesetztes Alkalimetallfluorid oder Tetraalkyl(C₁-C₁₈)-ammoniumfluorid, Oligo- oder Polyethylenglykoldimethylether, die 4 bis 150 Glykoleinheiten im Molekül enthalten, als Phasentransferkatalysator, gegebenenfalls in einem dipolaraprotischen Lösungsmittel, bei Temperaturen von etwa 140 bis etwa 200°C, insbesondere etwa 160 bis etwa 190°C, umsetzt.

An geeigneten Alkalimetallfluoriden kommen vor allem Kalium-, Rubidium- und Cäsiumfluorid in Frage, in Sonderfällen auch Lithium- oder Natriumfluorid. Vorzugsweise werden Kalium-, Rubidium- oder Cäsiumfluorid, oder Mischungen daraus eingesetzt. Mischungen aus Kaliumfluorid und Cäsiumfluorid sind bevorzugt. Besonders bevorzugt sind dabei solche Mischungen, die etwa 10 Gew.-% Cäsiumfluorid enthalten.

Beim erfindungsgemäßen Verfahren können auch sprühgetrocknete Alkalimetallfluoride eingesetzt werden.

Als Phasentransferkatalysatoren kommen quartäre Ammonium- oder Phosphoniumverbindungen, wie Tetraalkyl-(C₁-C₁₈)-ammoniumchloride, -bromide oder -fluoride, Tetraalkyl(C₁-C₁₈)-phosphoniumchloride oder -bromide, Tetraphenylphosphoniumchlorid oder -bromid oder (Phenyl)ₘ(alkyl(C₁-C₁₈))ₙ-phosphoniumchloride oder -bromide, wobei m = 1 bis 3, n = 3 bis 1 und m + n = 4 ist, in Frage. Bevorzugt sind hierbei Phosphoniumsalze, besonders bevorzugt Tetraalkyl(C₁-C₁₈)-phosphoniumbromide. Diese Stoffe werden in Mengen von etwa 0,5 bis etwa 10 Mol.-%, bevorzugt von etwa 1 bis etwa 5 Mol.-%, bezogen auf 2,4-Dichlor-5-fluorbenzonitril, eingesetzt. Verwendet man ein Tetraalkyl(C₁-C₁₈)-ammoniumfluorid als Fluoridsalz, so ist der Zusatz weiterer Phasentransferkatalysatoren nicht erforderlich, weil das Fluoridsalz bereits selbst einen solchen Katalysator darstellt.

Es können auch Oligo- oder Polyethylenglykoldimethylether als Phasentransferkatalysatoren eingesetzt werden. Die Zahl der Glykoleinheiten in diesen Verbindungen kann von etwa 4 (Tetraethylenglykoldimethylether) bis etwa 150 sein; bevorzugt werden jedoch Polyethylenglykoldimethylether eingesetzt, deren Polymerisationsgrad zwischen etwa 4 und etwa 25 ist. Bevorzugt werden die Glykolether zwischen etwa 10 und etwa 50 Gew.-%, bezogen auf das eingesetzte Fluorid, verwendet. Der besondere Vorteil der Verwendung dieser Verbindungen liegt darin, daß in der Regel der Einsatzmenge entsprechend weniger Lösungsmittel verwendet werden kann, weil die Glykolether bei der Reaktionstemperatur stets flüssig sind.

Überraschenderweise gelingt durch die Verwendung von Phasentransferkatalysatoren eine nahezu quantitative Umsetzung, während ohne diesen Zusatz kaum Umsätze von mehr als 70 %, dafür aber stark zunehmende Zersetzungen beobachtet werden können.

Das erfindungsgemäße Verfahren kann bei völliger Abwesenheit eines Lösungsmittels durchgeführt werden. Man kann jedoch auch in einem dipolar aprotischen Lösungsmittel, beispielsweise in Sulfolan (Tetramethylensulfon), Tetramethylensulfoxid, N,N-Diethylacetamid, N,N-Dimethylacetamid, N,N-Dimethylformamid, N-Methylpyrrolidon, Dimethylsulfoxid, Dimethylsulfon, Diphenylsulfoxid, Diphenylsulfon, Tetramethylharnstoff, Tetra-n-butylharnstoff, 1,3-Dimethylimidazolidin-2-on oder in Mischungen daraus umsetzen.

Das nach dem erfindungsgemäßen Verfahren erhaltene Produktgemisch wird im allgemeinen durch Filtration des Reaktionssalzes und - besonders bei Durchführung im technischen Maßstab - anschließendes Abdestillieren der leichtflüchtigen Bestandteile und Fraktionierung erhalten. Es ist auch eine direkte Fraktionierung aus dem Filtrat möglich. Ebenfalls möglich ist, das Rohgemisch mit Wasser zu versetzen und die leichtere Oberphase, die das Produkt enthält, abzutrennen. Durch Extraktion des Wassers kann eine vollständige Abtrennung des Produktes aus der Mutterlauge erhalten werden. Danach kann durch chromatographische oder destillative Trennung eine Reinigung erfolgen.

Das Verfahren kann bei Atmosphärendruck, Unter- oder Überdruck durchgeführt werden. Bevorzugt ist die Arbeitsweise bei leichtem Überdruck in einem geschlossenen Gefäß, um Verlust des leichtflüchtigen Produktes zu vermeiden, da dieses bei den Reaktionstemperaturen bereits einen erheblichen Dampfdruck besitzt. Man kann diesen Effekt dazu ausnutzen, das Produkt kontinuierlich während der Reaktion abzudestillieren, wodurch aber eine aufwendigere Apparatur und Steuerungstechnik (gleichmäßiger Rücklauf) benötigt wird. Eine nachfolgende Feinfraktionierung ist aber dennoch im allgemeinen erforderlich. Bei optimaler Einstellung der Reaktionsbedingungen, wie Katalysator, Konzentration, Temperatur und Salzmenge, erweist sich dieser zusätzliche Aufwand aber nicht als erforderlich, um hohe Ausbeuten und Raumleistungen zu erhalten.

Das Verfahren bietet einen Vorteil einer sehr konzentrierten Reaktionslösung, was aus Gründen der Wirtschaftlichkeit technisch erwünscht ist. Des weiteren erreicht man durch die erfindungsgemäße Verfahrensweise sehr hohe Umsätze, was ebenfalls zu hohen Raum-Zeit-Ausbeuten führt, wobei die Gesamtausbeuten dennoch gut bis sehr gut bleiben und gegenüber den bereits beschriebenen Verfahren erheblich verbessert sind. Gleichzeitig hat man bei dem erfindungsgemäßen Verfahren Handhabungsvorteile, da die Verwendung von sprühgetrocknetem Salz nicht zwingend erforderlich ist. Die Ausbeuten betragen etwa 65 bis etwa 85 % der Theorie, je nach Wahl des Katalysators, der Reaktionstemperatur und der Konzentration im Lösungsmittel.

Das als Ausgangsmaterial im erfindungsgemäßen Verfahren eingesetzte 2,4-Dichlor-5-fluorbenzonitril kann nach literaturbekannten Verfahren aus 2,4-Dichlor-5-fluorbrombenzol durch Brom-Cyan-Austausch (EP 433 124), aus 2,4-Dichlor-5-fluoranilin mittels Cyan-Sandmeyer-Reaktion (CN 1 031 074) oder aus 2,4-Dichlor-5-fluorbenzotrichlorid (EP 431 373) durch Ammonolyse hergestellt werden.

Die nachstehenden Beispiele erläutern das erfindungsgemäße Verfahren, ohne es darauf zu beschränken.

### Durchführungsbeispiele

In einigen Beispielen wurde der Reaktionsverlauf durch Eichung mittels internem Standard (inert unter den Reaktionsbedingungen) gaschromatographisch verfolgt. Dadurch läßt sich zu beliebigen Zeitpunkten exakt die gebildete Menge an Produkt bestimmen, die durch Aufarbeitung, wie im Beschreibungstext beschrieben, auf die in diesen Beispielen verzichtet wurde, erhalten werden könnte. Diese Ansätze könnten problemlos ohne Zusatz des inneren Standards in größerem Maßstab mit anschließender Aufarbeitung (bevorzugt Fraktionierung, aber auch Extraktion, Chromatographie) durchgeführt werden, wobei sogar im allgemeinen durch Verbesserung der Rührbedingungen bessere Ergebnisse, wie kürzere Reaktionszeiten, höhere Raumleistungen und bessere Ausbeuten, erhalten werden.
- Siedepunkt 2,4,5-Trifluorbenzonitril: 100 Torr/118°C
85 Torr/102°C
26 Torr/78°C
15 Torr/72°C

### Beispiel 1

13,6 g (0,22 Mol) Kaliumfluorid/Cäsiumfluorid (9:1) und 1,4 g Tetrabutylphosphoniumbromid werden in 50 g Sulfolan eingetragen. Danach werden etwa 10 g des Lösungsmittels im Vakuum abdestilliert (3 mbar/140°C). Dann gibt man bei 140°C 19,0 g (0,1 Mol) 2,4-Dichlor-5-fluorbenzonitril zu und erhitzt unter gutem Rühren für 11 h auf 190°C. Anschließend lassen sich in der Reaktionsmischung durch GC 13,2 g (84 %, 0,0839 Mol) 2,4,5-Trifluorbenzonitril nachweisen, die im Vakuum aus der Mischung entfernt und dann fraktioniert werden können.

### Beispiel 2

Man trägt unter Rühren in 20 g N-Methylpyrrolidon (NMP), 11,3 g (0,103 Mol) Rubidiumfluorid und 1,7 g Tetraoctylphosphoniumbromid (3 Mol-%, 3 mMol) ein und gibt zu der resultierenden Suspension 13,6 g (0,1 Mol) 2,4-Dichlor-5-fluorbenzonitril bei 100°C. Die Mischung wird in einem Autoklaven 14 h auf 210°C erhitzt und danach gaschromatographisch analysiert. Es lassen sich 10,9 g (69 %, 0,0692 Mol) 2,4,5-Trifluorbenzonitril durch Eichung gegen den inneren Standard nachweisen.

### Beispiel 3

Eine Reaktionsmischung aus 95,0 g (0,5 Mol) 2,4-Dichlor-5-fluorbenzonitril, 92,9 g (1,5 Mol) Kaliumfluorid/Cäsiumfluorid (9:1) und 20,3 g (8 Mol-%, 40 mMol) Hexadecyltributylphosphoniumbromid wird durch Andestillieren mit 40 g Toluol getrocknet. Nach vollständigem Entfernen des Toluols heizt man in einer geschlossenen Apparatur auf 210°C (15 h). Das abfiltrierte Reaktionssalz befreit man durch Waschen mittels Sulfolan von anhaftendem Produkt. Aus der ausreagierten Reaktionsmischung isoliert man durch Fraktionierung 52,2 g (66 %, 0,332 Mol) 2,4,5-Trifluorbenzonitril (RG (GC): > 99.5 %).

### Beispiel 4

136,2 g (2,2 Mol) Kaliumfluorid/Cäsiumfluorid (9:1) werden mit 6,0 g (1,5 Mol-%, 15 mMol) n-Butyltriphenylphosphoniumbromid und 300 g Sulfolan versetzt. Man destilliert an und gibt anschließend 190,0 g (1 Mol) 2,4-Dichlor-5-fluorbenzonitril zu und erhitzt auf 200°C. Nach 9 h ist die Umsetzung beendet. Das Reaktionssalz wird durch Filtration entfernt und die Mutterlauge fraktioniert. Man erhält 118,8 g (0,756 Mol, 76 %) 2,4,5-Trifluorbenzonitril, das bei 26 Torr (3,4 kPa)/78°C übergeht. Geringe Anteile nicht vollständig umgesetzter Benzonitrile (Zwischenläufe, Sdp. 80°-130°C/3,4 kPa) werden zusammen mit dem redestillierten Lösungsmittel wieder eingesetzt.

### Beispiel 5

Von einer Suspension aus 38,3 g (0,618 Mol) Kaliumfluorid/Cäsiumfluorid (9:1) und 1,3 g (1 Mol-%; 3 mMol) Tetraphenylphosphoniumbromid in 180 g N,N-Dimethylacetamid destilliert man etwa 20 g des Lösungsmittels ab. Man versetzt mit 57,0 g (0,3 Mol) 2,4-Dichlor-5-fluorbenzonitril und erhitzt in einem Glasautoklaven bei kräftiger Rührung 8 h auf 175°C. Danach lassen sich 33,7 g (71 %, 0,214 Mol) 2,4,5-Trifluorbenzonitril im Reaktionsgemisch durch Eichung gegen den internen Standard nachweisen.

### Beispiel 6

In 40 g Sulfolan werden 52,0 g (0,84 Mol) Kaliumfluorid/Cäsiumfluorid (9:1) eingetragen und 7,0 g Octadecyltrimethylammoniumchlorid (5 Mol-%, 20 mMol) zugesetzt. Man setzt bei 100°C 2,4-Dichlor-5-fluorbenzonitril (76,0 g, 0,4 Mol) zu und führt die Reaktion 5 h bei 205°C. Durch Eichung gegen inneren Standard lassen sich danach 41,4 g (66 %, 0,263 Mol) 2,4,5-Trifluorbenzonitril nachweisen.

### Beispiel 7

Zu 23,3 g (0,25 Mol) im Vakuum getrocknetem Tetramethylammoniumfluorid, das in Form eines farblosen Öls vorliegt, gibt man 30 ml wasserfreies N,N-Dimethylacetamid und 19,0 g (0,1 Mol) 2,4-Dichlor-5-fluorbenzonitril. Die Reaktionsmischung wird unter Argon 20 h bei 80°C gehalten und dann durch GC analysiert. Eichung gegen internen Standard zeigt 12,6 g (80 %, 0,0803 Mol) 2,4,5-Trifluorbenzonitril in der hellbraunen Lösung an.

### Beispiel 8

Man destilliert von einer Suspension aus 100 g N,N-Dimethylacetamid (DMAc), 50 g Polyethylenglykoldimethylether (n = 1000) und 38,7 g (0,625 Mol) Kaliumfluorid/Cäsiumflorid (9:1) etwa 70 g DMAc ab, gibt 47,5 g (0,25 Mol) 2,4-Dichlor-5-fluorbenzonitril zu und erhitzt in einem Glasautoklaven bei guter Rührung 10 h auf 200°C. Danach destilliert man die leichtflüchtigen Bestandteile (Produkt, DMAc, Nebenprodukte) aus der Reaktionsmischung ab (30 Torr/50°C-130°C) und unterwirft das Rohgemisch einer Feinfraktionierung. Man erhält nach dem Lösungsmittel bei 85 Torr/102°C 30,1 (28,3) g (72 %, 0,180 Mol) 2,4,5-Trifluorbenzonitril (RG (GC): ca. 94 %) Reineres Produkt erhält man bei Vergrößerung der Ansätze.

### Beispiel 9

Man gibt zu 50 g Sulfolan 19,4 g (0,3 Mol) Kaliumfluorid/Cäsiumfluorid (5:1) und destilliert etwa 20-25 g Lösungsmittel im Vakuum ab. Zum Sumpf gibt man 7,9 g wasserfreien Tetraethylenglykoldimethylether und 19,0 g (0,1 Mol) 2,4-Dichlor-5-fluorbenzonitril und erhitzt 15 h auf 160°C. Anschließend zeigt gaschromatographische Analyse (Eichung gegen inneren Standard), daß sich in der viskosen Reaktionsmischung 10,1 g (64 %, 0,0643 Mol) Produkt befinden.

### Beispiel 10 (Vergleichsbeispiel)

In 60 g Sulfolan suspendiert man 13,6 g (0,22 Mol) Kaliumfluorid/Cäsiumfluorid (9:1) und destilliert im Vakuum 10 g des Lösungsmittels ab. Nach Zugabe von 19,0 g (0,1 Mol) 2,4-Dichlor-5-fluorbenzonitril erhitzt man 20 h bei 200°C. Die durch Eichung gegen internen Standard bestimmten Ausbeuten (GC) an 2,4,5-Trifluorbenzonitril erreichen nach 14-16 h ein Maximum mit etwa 50 % der Theorie (die Ausbeuten nehmen im weiteren Verlauf der Umsetzung wieder ab). Der Umsatz beträgt nach der Reaktionszeit etwa 85-90 %.

### Beispiel 11 (Hydrolysebeispiel)

Zu 30 g 75 prozentiger Schwefelsäure tropft man bei 180°C 20 g (0,127 Mol) 2,4,5-Trifluorbenzonitril in 1 h zu. Die 100°C heiße Lösung wird auf 50 g Eis gegeben und bei 0°C abgesaugt. Das Produkt wäscht man dreimal mit 30 g Eiswasser und trocknet bei 50°C im Vakuum. Durch Extraktion der Mutterlaugen mit Dichlormethan, Trocknen über Magnesiumsulfat und Entfernen des Lösungsmittels erhält man 1,9 g (9 % 0,011 Mol) leicht gelbliche Kristalle. Als Hauptmenge ergeben sich 19,3 g 2,4,5-Trifluorbenzoesäure (86 %, 0,110 Mol) in Form farbloser Kristalle. Die Gesamtausbeute beträgt 95 % (RG (GC, HPLC) > 99 %; Fp. 98,3-99,5°C). Durch Umkristallisieren aus Wasser kann man Produkt vom Schmelzbereich 100,6-101,8°C erhalten.

## Patentansprüche

1. Verfahren zur Herstellung von 2,4,5-Trifluorbenzonitril, dadurch gekennzeichnet, daß man 2,4-Dichlor-5-fluorbenzonitril mit etwa 105 bis etwa 150 Mol-% eines Alkalimetallfluorids oder eines Tetraalkyl-(C₁-C₁₈)-ammoniumfluorids oder Mischungen aus solchen Fluoriden pro auszutauschendem Chloratom in Gegenwart von 0,5 bis 10 Mol-%, bezogen auf 2,4-Dichlor-5-fluorbenzo nitril eines Tetraalkyl-(C₁-C₁₈₎-phosphoniumchlorids oder -bromids, Tetraphenylphosphoniumchlorids oder -bromids, oder (Phenyl)ₘ(alkyl(C₁-C₁₈))ₙ-phosphoniumchlorids oder -bromids, wobei m = 1 bis 3, n = 3 bis 1 und m + n = 4 ist, oder 5 bis 100 Gew.-%, bezogen auf eingesetztes Alkalimetallfluorid oder Tetraalkyl(C₁-C₁₈)-ammoniumfluorid, Oligo- oder Polyethylenglykoldimethylether, die 4 bis 150 Glykoleinheiten im Molekül enthalten, als Phasentransferkatalysator, gegebenenfalls in einem dipolar-aprotischen Lösungsmittel, bei Temperaturen von etwa 140 bis etwa 200°C umsetzt.

2. Verfahren nach Anspruch 1, dadurch gekennzeichnet, daß man mit etwa 110 bis etwa 120 Mol-% eines Alkalimetallfluorids oder Tetraalkyl(C₁-C₁₈)-ammoniumfluorids oder Mischungen aus solchen Fluoriden umsetzt.

3. Verfahren nach mindestens einem der Ansprüche 1 und 2, dadurch gekennzeichnet, daß man bei Temperaturen von etwa 160 bis etwa 190°C umsetzt.

4. Verfahren nach mindestens einem der Ansprüche 1 bis 3, dadurch gekennzeichnet, daß man als Alkalimetallfluoride Kalium-, Rubidium- oder Cäsiumfluorid oder Mischungen daraus einsetzt.

5. Verfahren nach mindestens einem der Ansprüche 1 bis 4, dadurch gekennzeichnet, daß man als Alkalimetallfluoride Mischungen aus Kaliumfluorid und Cäsiumfluorid, die etwa 10 Gew.-% Cäsiumfluorid enthalten, einsetzt.

6. Verfahren nach mindestens einem der Ansprüche 1 bis 3, dadurch gekennzeichnet, daß man als Tetraalkylammoniumfluoride Tetramethylammoniumfluorid, Tetra-n-butylammoniumfluorid und/oder Tetra-n-octylammoniumfluorid einsetzt.

7. Verfahren nach mindestens einem der Ansprüche 1 bis 6, dadurch gekennzeichnet, daß man bei Unter-, Über- oder Atmosphärendruck umsetzt.

8. Verfahren nach mindestens einem der Ansprüche 1 bis 7, dadurch gekennzeichnet, daß man das entstandene 2,4,5-Trifluorbenzonitril während der Reaktion kontinuierlich abdestilliert.

## Claims

1. A process for the preparation of 2,4,5-trifluorobenzonitrile, which comprises reacting 2,4-dichloro-5-fluorobenzonitrile with approximately 105 to approximately 150 mol % of an alkali metal fluoride or of a tetraalkyl(C₁-C₁₈)ammonium fluoride or mixtures of such fluorides per chlorine atom to be exchanged, at temperatures from approximately 140 to approximately 200°C, in the presence of 0.5 to 10 mol %, relative to 2,4-dichloro-5-fluorobenzonitrile, of a tetraalkyl(C₁-C₁₈)phosphonium chloride, tetraalkyl(C₁-C₁₈)phosphonium bromide, tetraphenylphosphonium chloride, tetraphenylphosphonium bromide, (phenyl)ₘ(alkyl(C₁-C₁₈))ₙ-phosphonium chloride or (phenyl)ₘ(alkyl(C₁-C₁₈))ₙ-phosphonium bromide, where m is 1 to 3, n is 3 to 1 and m+n is 4, or 5 to 100 % by weight, relative to alkali metal fluoride or tetraalkyl(C₁-C₁₈)ammonium fluoride employed, of oligo- or polyethylene glycol dimethyl ethers which contain 4 to 150 glycol units in the molecule, as phase transfer catalyst, if appropriate in a dipolar aprotic solvent.

2. The process as claimed in claim 1, wherein the reaction is carried out with approximately 110 to approximately 120 mol % of an alkali metal fluoride or tetraalkyl(C₁-C₁₈) ammonium fluoride or mixtures of such fluorides.

3. The process as claimed in at least one of claims 1 and 2, wherein the reaction is carried out at temperatures from approximately 160 to approximately 190°C.

4. The process as claimed in at least one of claims 1 to 3, wherein potassium fluoride, rubidium fluoride or cesium fluoride or mixtures of these are employed as alkali metal fluorides.

5. The process as claimed in at least one of claims 1 to 4, wherein mixtures of potassium fluoride and cesium fluoride which contain approximately 10 % by weight of cesium fluoride are employed as alkali metal fluorides.

6. The process as claimed in at least one of claims 1 to 3, wherein tetramethylammonium fluoride, tetra-n-butylammonium fluoride and/or tetra-n-octylammonium fluoride are employed as tetraalkylammonium fluorides.

7. The process as claimed in at least one of claims 1 to 6, wherein the reaction is carried out under subatmospheric, superatmospheric or atmospheric pressure.

8. The process as claimed in at least one of claims 1 to 7, wherein the resulting 2,4,5-trifluorobenzonitrile is continuously distilled off during the reaction.

## Revendications

1. Procédé de préparation de 2,4,5-trifluorobenzonitrile, caractérisé en ce que l'on fait réagir le 2,4-dichloro-5-fluorobenzonitrile avec d'environ 105 à environ 150 % en moles d'un fluorure de métal alcalin ou d'un fluorure de tétra(alkyl en C₁-C₁₈)ammonium ou de mélanges de tels fluorures, par atome de chlore à échanger, en présence de 0,5 à 10 % en moles, par rapport au 2,4-dichloro-5-fluorobenzonitrile d'un chlorure ou bromure de tétra(alkyl en C₁-C₁₈)phosphonium, d'un chlorure ou d'un bromure de tétraphénylphosphonium ou de bromure ou de chlorure de phényl)ₘ(alkyl en C₁-C₁₈)ₙphosphonium, où m = 1 à 3, n = 3 à 1 et m+n = 4, ou de 5 à 100 % en poids, par rapport au fluorure de métal alcalin utilisé ou au fluorure de tétra(alkyl en C₁-C₁₈)ammonium, de diméthyléther d'oligo- ou de polyéthylène glycol, qui contient de 4 à 150 unités glycol dans la molécule, en tant que catalyseur de transfert de phase, éventuellement dans un solvant dipolaire-aprotique, à une température de 140 à environ 200 °C.

2. Procédé selon la revendication 1, caractérisé en ce que l'on fait réagir avec d'environ 110 à environ 120 % en moles d'un fluorure de métal alcalin ou de fluorure de tétra(alkyl en C₁-C₁₈)ammonium ou de mélanges de tels fluorures.

3. Procédé selon au moins l'une de revendications 1 et 2, caractérisé en ce que l'on met en oeuvre la réaction à une température d'environ 160 à environ 190 °C.

4. Procédé selon au moins l'une des revendications 1 à 3, caractérisé en ce que l'on utilise du fluorure de potassium de rubidium ou de césium ou leurs mélanges comme fluorures de métaux alcalins.

5. Procédé selon au moins l'une des revendications 1 à 4, caractérisé en ce que l'on utilise comme fluorures de métaux alcalins des mélanges de fluorure de potassium et de fluorure de césium, contenant environ 10 % en poids de fluorure de césium.

6. Procédé selon l'une des revendications 1 à 3, caractérisé en que l'on utilise comme fluorures de tétraalkylammonium le fluorure de tétraméthylammonium, le fluorure de tétra-n-butylammonium et/ou le fluorure de tétra-n-octylammonium.

7. Procédé selon au moins l'une des revendications 1 à 6, caractérisé en ce que l'on met en oeuvre la réaction sous un défaut de pression, une surpression ou à la pression atmosphérique.

8. Procédé selon au moins l'une des revendications 1 à 7, caractérisé en ce que l'on chasse en continu au cours de la réaction le 2,4,5-trifluorobenzonitrile formé par distillation.
